(19) Europäisches Patentamt
European Patent Office
Office européen des brevets

(11) Numéro de publication: **0 008 973**
**B1**

(12) # FASCICULE DE BREVET EUROPEEN

(45) Date de publication du fascicule du brevet: **29.09.82**

(21) Numéro de dépôt: **79400573.6**

(22) Date de dépôt: **14.08.79**

(51) Int. Cl.³: **C 07 C 59/68,**
C 07 C 51/06,
C 07 C 103/178,
C 07 C 102/08,
C 07 D 295/18,
C 07 C 121/38,
C 07 C 120/00,
C 07 C 69/16,
C 07 C 67/14,
C 07 C 67/287,
C 07 C 102/00

(54) **Procédé de fabrication des acides phénoxylactiques, de leurs dérivés et produits obtenus.**

(30) Priorité: **18.08.78 FR 7824116**

(43) Date de publication de la demande:
**19.03.80 Bulletin 80/6**

(45) Mention de la délivrance du brevet:
**29.09.82 Bulletin 82/39**

(84) Etats contractants désignés:
**CH DE FR GB**

(73) Titulaire: **SUCRERIES DU SOISSONNAIS ET COMPAGNIE SUCRIERE Société anonyme dite:**
**336, rue Saint-Honoré**
**F-75001 Paris (FR)**

(72) Inventeur: **Anatol, Jésus**
**27, rue de Longchamp**
**F-75116 Paris (FR)**
Inventeur: **Clenet, Yves**
**12 Square Henry Paté**
**F-75016 Paris (FR)**
Inventeur: **Bourdiau, Georges**
**90 Avenue de la Division Leclerc**
**F-93350 Le Bourget (FR)**

(74) Mandataire: **Rinuy, Guy et al,**
**14, Avenue de la Grande Armée**
**F-75017 Paris (FR)**

(56) Documents cités:
US - A - 2 448 873
US - A - 2 518 456
US - A - 3 374 245

JOURNAL OF THE AMERICAN CHEMICAL SOCIETY, vol. 77, no. 21, 11 novembre 1955 B. J. LUDWIG et al.: "Synthesis of -(4-Hydroxy-2-methylphenoxy)-lactic Acid, a Metabilite of Mephenesin" pages 5751—5753.

(56) Documents cités
JOURNAL OF THE AMERICAN CHEMICAL SOCIETY, vol 73, septembre 6, 1951, no. 9, American Chemical Society, Easton PA 18042 US SCHREYER: "Synthesis of 3-Amino-1,2-propanediol and 2,3-diamino-1-propanol.

Comprehensive Organic Chemistry. Barton & Ollis. Pergamon Press (1979) Vol. II. pp. 780—781.

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 008 973 B1

Courier Press, Leamington Spa, England.

Procédé de fabrication des acides phénoxylactiques, de leurs dérivés et produits obtenus

La présente invention concerne l'obtention d'une classe générale de composés chimiques, à savoir celle des acides phénoxylactiques et de leurs dérivés ainsi que les produits nouveaux en résultant.

Les composés visés par l'invention répondant à la formule générale:

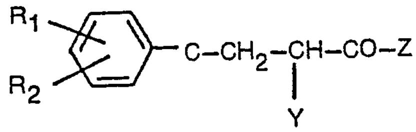

(A)

où

$R_1$ et $R_2$ sont identiques ou différents et peuvent représenter des atomes d'hydrogène, des restes méthyle, des groupes méthoxy ou des halogènes;

Y représente un groupement —OH ou —O—CO—$R_3$ avec $R_3$ représentant soit un reste aliphatique éventuellement substitué par un halogène, soit un reste aromatique éventuellement substitué sur le noyau par un ou plusieurs groupements méthyle, méthoxy, halogéno, soit un reste arylaliphatique saturé ou insaturé, soit enfin un hétérocycle;

Z représente le groupement —OH ou un groupement

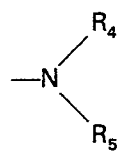

avec $R_4$ représentant un radical alkyle ou aryle et

$R_5$ de l'hydrogène ou un radical alkyle, —$NR_4R_5$ pouvant aussi représenter le radical morpholinyle.

Ainsi:

—Dans le cas particulier où Z et Y représentent OH, les composés de l'invention sont les acides phénoxylactiques eux-mêmes de formule générale:

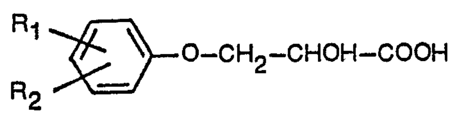

($A_1$)

où $R_1$ et $R_2$ ont la signification donnée ci-dessus;

—Dans le cas particulier où Z représente le groupe OH et Y représente le groupe —O—CO—$R_3$, les composés de l'invention sont les esters en position 2 des acides ($A_1$) ci-dessus répondant à la formule générale:

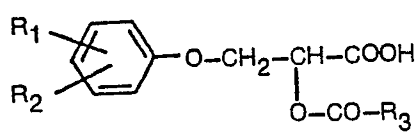

($A_2$)

où $R_1$ et $R_2$ ont la signification donnée ci-dessus;

—Dans le cas particulier où Y représente OH et Z représente le groupement

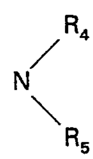

tel que défini ci-dessus, les composés de l'invention sont les amides secondaires et tertiaires des acides ($A_1$) ci-dessus, répondant à la formule générale:

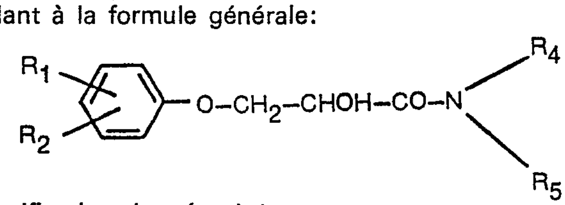

($A_3$)

où $R_1$ et $R_2$ ont la signification donnée ci-dessus;

—et enfin dans le cas particulier où Y représente le groupement —O—CO—$R_3$ tel que défini ci-dessus et Z représente le groupement $NR_4R_5$ tel que défini ci-dessus, les composés de l'invention sont les esters-amides dérivés de $A_3$ répondant à la formule générale:

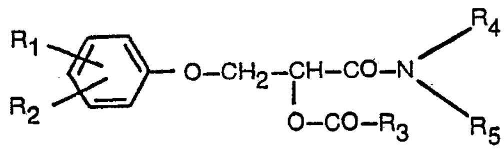

($A_4$)

où $R_1$ et $R_2$ ont la signification donnée précédemment.

Le procédé selon l'invention est essentiellement caractérisé par le fait qu'il consiste à soumettre une cyanhydrine de formule:

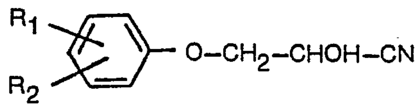

(A)

(où $R_1$ et $R_2$ ont la signification donnée ci-dessus) à une réaction de RITTER (réaction avec du tert.butanol ou de l'isobutène en présence d'un acide qui est en général l'acide sulfurique) pour obtenir le composé de formule:

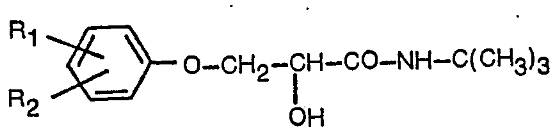

($A_{3a}$)

à hydrolyser ce dernier pour obtenir le composé correspondant de formule:

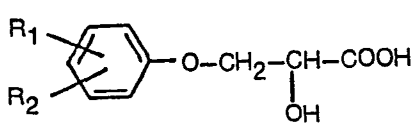

($A_1$)

lequel est alors soumis aux réactions classiques d'obtention de l'ester-acide de formule:

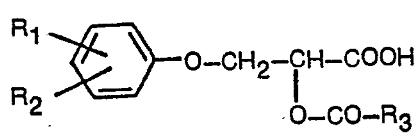

($A_2$)

de l'amide de formule:

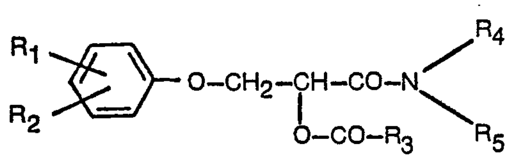

($A_4$)

ou de formule:

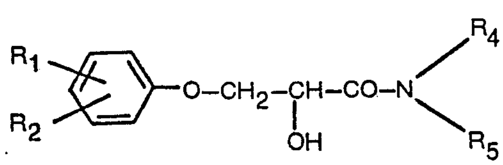

($A_3$)

où $R_4$ et $R_5$ ont la signification donnée ci-dessus.

La cyanhydrine de départ servant à la mise en oeuvre du procédé de l'invention est obtenue à partir du phénoxy acétaldéhyde correspondant en utilisant les méthodes usuelles d'obtention d'une cyanhydrine à partir d'un aldéhyde.

Ces cyanhydrines (phénoxy-3 lactonitriles) constituent des produits nouveaux.

Soumises aux conditions de la réaction de Ritter comme indiqué plus haut, ces cyanhydrines conduisent, avec de bons rendements, aux tertiobutylamides ($A_{3a}$) des acides phénoxy-3 lactiques dont aucun représentant n'est cité dans la littérature à la connaissance de la Demanderesse.

L'hydrolyse de ces tertiobutylamides permet d'obtenir les acides phénoxy-3 lactiques correspondants ($A_1$).

Disposant de ces acides, l'homme de l'art possède alors un moyen permettant d'obtenir les acides O-acyl phénoxy-3 lactiques ($A_2$) correspondants dont tous les représentants sont des produits nouveaux.

Ces dérivés traités par des agents chlorurants donnent les chlorures d'acides correspondants lesquels, soumis à l'action des amines, peuvent être facilement transformés en esters-amides correspondants ($A_4$). Par ailleurs, si l'on soumet ces derniers composés à une désacylation par hydrolyse sélective, on obtient les différents alpha-hydroxyamides des acides phénoxy-3 lactiques.

Ces hydroxyamides peuvent à leur tour être O-acylés à l'aide des chlorures d'acides: $R_3$—CO—Cl. Ceci constitue une voie d'accès intéressante aud dérivés $A_4$ qui peuvent ainsi être obtenus:

—soit à partir des esters en position 2 des acides phénoxyactiques;

—soit à partir des hydroxyamides ($A_3$) obtenus par exemple à partir d'un composé $A_4$ facilement accessible.

Certains composés $A_{4a}$ dans lesquels $R_4$=$(CH_3)_3C$ et $R_5$=H peuvent évidemment être obtenus beaucoup plus directement à partir des hydroxyamides ($A_{3a}$).

Le schéma réactionnel suivant illustre le mode d'obtention des composés ci-dessus.

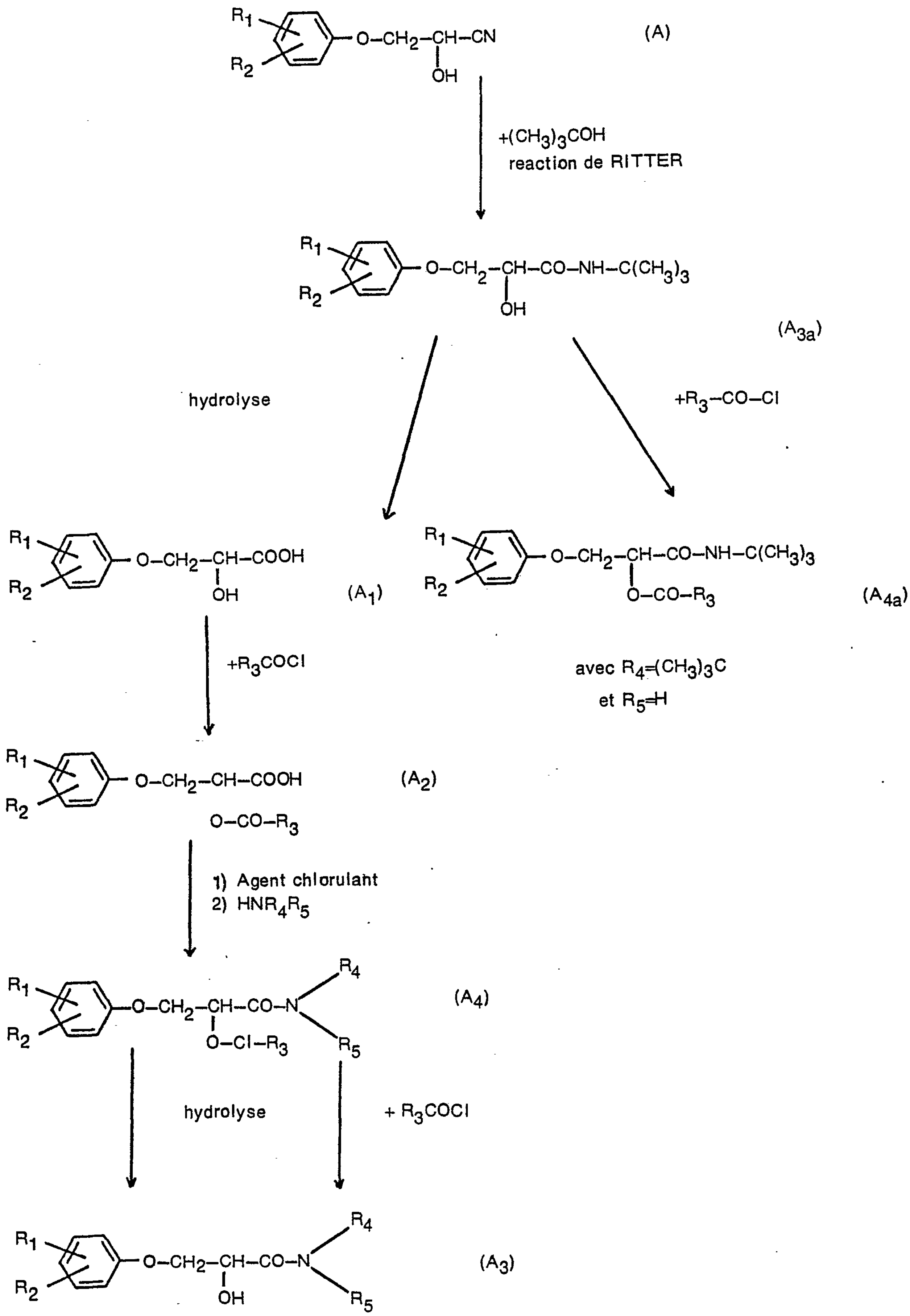


(A)

($A_{3a}$)

($A_1$)

($A_{4a}$)

($A_2$)

($A_4$)

($A_3$)

Les exemples suivants sont donnés à titre illustratif, nullement limitatif. Dans ces exemples, les composés accompagnés d'un astérisque (*) sont des composés connus. Ce sont des acides bêta-phenoxylactiques qui ont été généralement obtenus par la méthode de C. Frederick Koelsh (J.Am.Chem.Soc. 52, 2430—6 (1930)) à partir de l'acide bêta-chloro-lactique et du phénol correspondant en présence de soude.

Les points de fusion ont été pris au banc Köfler.

Les chromatographies sur couches minces (C.C.M.) ont été réalisées sur plaques de gel de silice sur support plastique, éluées par la méthode ascendante et révélées avec un mélange sulfopermanganique. Les éluants utilisés sont:

A) chloroforme seul; B) chloroforme/méthanol (99/1); C) chloroforme/méthanol (95/5); D) chloroforme/méthanol (90/10).

Exemple 1

*Hydroxy-2 phénoxy-3 propanenitrile*

A: $R_1$=$R_2$=H) "Composé N° 1"

Dans un tricol de 1 litre avec agitation mécanique, thermomètre et ampoule à brome, on introduit 136 g (1 mole) de phosphate monopotassique et 408 ml d'eau. A la température ambiante, on ajoute 136 g (1 mole) de phénoxyacétaldéhyde. Un très beau précipité se forme aussitôt et la température s'élève légèrement.

En refroidissant au bain de glace, ou ajoute par l'ampoule à brome une solution de 65 g (1 mole) de cyanure de potassium dans 130 ml d'eau en veillant à ce que la température du mélange ne dépasse pas 15°C. L'addition prend environ 30 minutes. Il se forme une huile qui est émulsionnée par l'agitation et qui commence à précipiter peu après la fin de l'addition. On continue l'agitation dans le bain de glace pendant 30 minutes puis filtre les cristaux, les lave à l'eau jusqu'à neutralité et les sèche à poids constant. Le produit obtenu fondant à environ 56°C est recristallisé dans 10 volumes de tétrachlorure de carbone. On obtient ainsi un produit fondant à 59°C.

C.C.M: éluant C une seule tache Rf=0,3

Analyse: $C_9H_9NO_2$ M=163,18

| | | | |
|---|---|---|---|
| Calc.%: | C: 66,24 | H: 5,56 | N: 8,58 |
| Tr%: | 66,18 | 5,62 | 8,73 |

De façon similaire, on a obtenu les cyanhydrines (A) réunies dans le tableau I:

TABLEAU I: Phénoxy-3 lactonitriles A:

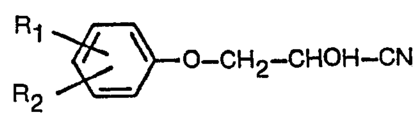

| Composé N° | $R_1$ | $R_2$ | p.f. °C | Recristallisation |
|---|---|---|---|---|
| 1 | H | H | 60 | $CCl_4$ 10 vol. |
| 2 | o-$CH_3$ | H | 60 | $CCl_4$ |
| 3 | p-$CH_3$ | H | 64 | $CCl_4$ |
| 4 | o-$CH_3$ | o'-$CH_3$ | 80 | $CCl_4$ |
| 5 | o-$OCH_3$ | H | 88 | $CCl_4$ 7,5 vol. |
| 6 | p-$OCH_3$ | H | 63 | Trichloréthylène 2 vol. |
| 7 | p-Cl | H | 72–5 | $CCl_4$ |

Exemple 2

*N-tert.-butyl hydroxy-2 p-tolyloxy-3 propanamide*

($A_3$; $R_1$=p—$CH_3$, $R_2$=$R_5$=H, $R_4$=$(CH_3)_3C$) Composé N° 10

Dans un ballon de 500 ml avec agitation, réfrigérant, thermomètre et ampoule d'introduction, à un mélange de 20,4 g (0,115 mole) de p-tolyloxy-3 lactonitrile et 120 ml de tert.-butanol, on ajoute 50 ml d'éthérate de trifluorure de bore puis maintient dans un bain d'eau à 50°C pendant 2 heures.

On coule ensuite sur 500 ml d'eau froide, sépare le produit gommeux obtenu et le fait cristalliser dans 100 ml d'éther isopropylique. Après filtration et séchage, le produit obtenu est recristallisé dans 12 volumes de mélange éthanol (2 vol.)-éther isopropylique (10 vol.). F=141—2°C.

C.C.M.: éluent à une seule tache $R_f$=0,4

Analyse: $C_{14}H_{21}NO_3$ M=251,34

| | | | |
|---|---|---|---|
| Calculé %: | C: 66,91 | H: 8,42; | N: 5,57 |
| Trouvé%: | 66,66 | 8,54 | 5,55 |
| | 66,61 | 8,40 | |

Spectre I.R. (pastille KBr à 1%): bandes à 3375 (CO—*NH*—), 3200 (OH), 1640—60 $cm^{-1}$ (*CO*—NH).

Les N-tert.-butyl hydroxy-2 aryloxy-3 propanamides ($A_{3a}$) réunis dans le tableau suivant ont été obtenus de façon similaire.

TABLEAU II: N-tert.-butyl hydroxy-2 aryloxy-3 propanamides ($A_{3a}$)

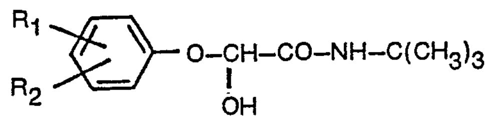

| Composé N° | $R_1$ | $R_2$ | F°C | Recristallisation | C.C.M. eluant Rf |
|---|---|---|---|---|---|
| 8 | H | H | 123 | $CCl_4$ 3 vol. | B 0,36 |
| 9 | o-$CH_3$ | H | 80 | hexane 10 vol. | B 0,45 |
| 10 | p-$CH_3$ | H | 141 | EtOH 2 vol. + éther isopropylique 10 vol. | A 0,40 |
| 11 | o-$CH_3$ | o'-$CH_3$ | 130 | AcOEt 4 vol. | A 0,38 |
| 12 | o-$OCH_3$ | H | 96 | Ether isopropylique 4 vol. | C 0,27 |
| 13 | p-$OCH_3$ | H | 100—4 | Ether isopropylique 5 vol. | C 0,30 |
| 14 | p-Cl | H | 130 | MeOH 1 vol. + éther isopropylique 4,5 vol. | D 0,75 |

Exemple 3

*Acide hydroxy-2 p-chlorophénoxy-3- propanoïque*

($A_1$; $R_1$=p—Cl, $R_2$=H) Composé N° 21

Un mélange de 5,43 g (0,02 mole) de N-tert.-butyl hydroxy-2 p-chlorophénoxy-3 propanamide dans 16,3 ml d'eau, 16,3 ml d'acide chlorhydrique concentré (d=1,18) et 5,4 ml d'acide acétique est porté à reflux pendant 3 h 45 jusqu'à ce que la solution devienne limpide. Au refroidissement, le produit cristallise. Il est filtré, lavé à l'eau et séché. Il est recristallisable dans l'eau (40 vol.). Cristaux en paillettes F=138°C.

C.C.M. éluant C Rf=0,48

Analyse: $C_9H_9ClO_4$ M=216,62

| | | |
|---|---|---|
| Calculé%: | C: 49,90 | H: 4,19 |
| Trouvé%: | 50,02 | 4,08 |

Spectre I.R. (pastille KBr à 1%) : bandes à 3350 (OH) et 1715 $cm^{-1}$ (*CO*OH).

Les acides aryloxy-3 lactiques ($A_1$) obtenus de façon similaire sont reassemblés dans le Tableau III.

TABLEAU III: Acides aryloxy-3 lactiques ($A_1$):

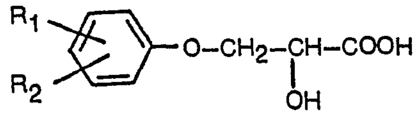

| Composé N° | $R_1$ | $R_2$ | F°C | Recristallisation | C.C.M. eluant Rf |
|---|---|---|---|---|---|
| 15* | H | H | 160 | eau 4 vol. | D 0,58 |
| 16* | o-$CH_3$ | H | 145 | eau 10 vol. | C 0,53 |
| 17 | p-$CH_3$ | H | 153 | eau 10 vol. | D 0,26 |
| 18 | o-$CH_3$ | o'-$CH_3$ | 137 | eau 10 vol. | D 0,75 |
| 19* | o-$OCH_3$ | H | ∼90 | huile cristallisant mal | D 0,34 |
| 20 | p-$OCH_3$ | H | 160 | EtOH 6 vol. | D 0,44 |
| 21 | p-Cl | H | 137 | eau 40 vol. | C 0,42 |

Exemple 4

*Acide acétoxy-2 (p-chlorophénoxy)-3 propanoïque*

($A_2$; $R_1$=p-Cl, $R_2$=H, $R_3$=$CH_3$) Composé No 22

A 38, 97 g (0.18 mole) d'acide hydroxy-2 p-chlorophénoxy-3 propanoïque placés dans un ballon de 250 ml surmonté d'un réfrigérant muni d'une garde à chlorure de calcium, on ajoute 58,5 ml de chlorure d'acétyle et maintient dans un bain à 45°C pendant 4 h. On concentre à l'évaporateur rotatif, reprend le résidu avec du toluène que l'on chasse pour entraîner l'excès de chlorure d'acétyle. Le résidu est dissous dans 180 ml d'une solution aqueous de bicarbonate de sodium à 10%. La solution obtenue est traitée au noir animal à température ambiante pendant 15 minutes puis filtrée sur papier. La solution incolore obtenue est acidifiée à pH=1 avec de l'acide chlorhydrique 2N.

Le précipité obtenu est filtré, lavé à l'eau et séché.

F=51,5—52,5° (capillaire).

Il s'agit d'un monohydrate qui, séché au dessiccateur sous vide, se transforme en huile.

C.C.M.: éluant C Rf=0,31

Analyse: $C_{11}H_{11}ClO_5$: 1 $H_2O$ M=276,67

| | | |
|---|---|---|
| Calc.%: | 47,75 | H: 4,73 |
| Tr.%: | 47,74 | 4,52 |
| | 47,95 | 4,51 |

Spectre I.R. (pastille KBr à 1%): bande à 1750 $cm^{-1}$ (*C*OOH).

Exemple 5

De la même façon, on a obtenu:

*l'acide acetoxy-2 phénoxy-3 propanoïque*

($A_2$; $R_1$=$R_2$=H, $R_3$=$CH_3$) Composé No 23 qui a également été purifié par l'intermédiaire de son sel de sodium obtenu à l'aide de bicarbonate de sodium. F=109°C.

C.C.M.: éluant D Rf=0,71

Analyse: $C_{11}H_{12}O_5$ M=224,21

| | | |
|---|---|---|
| Calc. %: | C: 58,93 | H: 5,39 |
| Tr%: | 59,07 | 5,42 |
| | 59,04 | 5,46 |

Spectre I.R. (pastille KBr à 1%): bandes à 1760 et 1710 $cm^{-1}$ (CO acide et CO ester).

Exemple 6

a) *chlorure de phénoxy-3 acétoxy-2 propionyle*

A 22,42 g (0,1 mole) d'acide acétoxy-2 phénoxy-3 propanoïque, on ajoute 24 g de chlorure de thionyle et chauffe à reflux pendant 2 h. L'excès de chlorure de thionyle est chassé à l'évaporateur rotatif et le résidu est distillé sous azote. $E_{14}$=163—8°C.

b *N-morpholino acétoxy-2 phénoxy-3 propanamide*
($A_4$; $R_1$=$R_2$=H, $R_3$=$CH_3$, $NR_4R_5$=morpholinyl)
Composé no 45.

A 1,73 ml (0,02 mole) de morpholine en solution dans 25 ml d'éther isopropylique, on ajoute 2,42 g (0,01 mole) de chlorure d'acétoxy-2 phénoxy-3 propionyle en solution dans 15 ml d'éther isopropylique. La précipitation de chlorhydrate de morpholine est immédiate. Après 4 h d'agitation à température ambiante, le précipité est filtré et lavé avec un peu d'éther isopropylique. Le filtrat est mis à sec. Le résidu obtenu est dissous dans du chloroforme, lavé à eau et concentré à sec. Le résidu est recristallisé dans un mélange de 1 volume de tétrachlorure de carbone et 9 volumes d'éther isopropylique. F=89°C.

C.C.M.: éluant B Rf=0,55
Analyse: $C_{15}H_{19}NO_5$ M=293,33

| | | | |
|---|---|---|---|
| Calc. %: | C: 61,42 | H: 6,53, | N: 4,78 |
| Tr.%: | 61,33 | 6,74 | 4,87 |

Spectre I.R. (pastille KBr à 1%): bandes à 1745 (COester) et 1655 $cm^{-1}$ (CO amide).

## Exemple 7

*N-isopropyl hydroxy-2 (p-chlorophénoxy)-3 propanamide*
($A_3$: $R_1$=p-Cl, $R_2$=$R_5$=H, $R_4$= $(CH_3)_2CH$) composé no 24

A 3 g (0.01 mole) de N-isopropyl acétoxy-2 (p-chlorophénoxy)-3 propanamide, on ajoute 9 ml d'alcool et 4 ml d'une solution de soude à 10% sous agitation magnétique. On obtient une solution jaune que l'on chauffe au bain-marie pendant 1/2 heure. Au refroidissement, le produit cristallise. Il est filtré, lavé à l'eau, séché et finalement recristillisé dans 5 volumes d'acétate d'éthyle. F=117°C.

C.C.M.: éluant D Rf=0,50
Analyse: $C_{12}H_{16}ClNO_3$ M=252,72

| | | | |
|---|---|---|---|
| Calc.%; | C 55,93 | H: 6,26 | N 5,43 |
| Tr.%: | 55,76 | 6,18 | 5,40 |

Spectre I.R. (pastille KBr à 1%): bandes à 3370 (OH), 3320 (NH), 1640—60$cm^{-1}$ (*CO*NH).

## Exemple 8

*N-isopropyl hydroxy-2 phénoxy-3 propanamide*
($A_3$; $R_1$=$R_2$=$R_5$=H, $R_4$=$(CH_3)_2CH$) Composé No 25

Ce produit est obtenu comme pour l'exemble 7 à partir de 2,65 g de N-isopropyl acétoxy-2 phénoxy-3 propanamide. F=68—70°C (éther isopropylique 4 volumes).

C.C.M.: éluant B Rf=0,33
Analyse: $C_{12}H_{17}NO_3$ M=223,27

| | | | |
|---|---|---|---|
| Calc. %: | C: 64,55 | H: 7,67 | N 6,27 |
| Tr%: | 64,29 | 7,79 | 6,22 |
| | 64,36 | 7,85 | |

Spectre I.R. (pastille KBr à 1%): bandes à 3380 (OH), 3320 (NH), 1630 $cm^{-1}$ (*CO*NH).

## Exemple 9

*N-isopropyl (triméthoxy-3,4,5 benzoyloxy)-2 (p-chlorophénoxy)-3 propanamide*
($A_4$; $R_1$=p-Cl, $R_2$=$R_5$=H, $R_3$= 3,4,5 $(MeO)_3C_6H_2$, $R_4$=$(CH_3)_2CH$) Composé No 110

A 5,15 g (0,02 mole) de N-isopropyl hydroxy-2 (p-chlorophénoxy)-3 propanamide, 50 ml de toluène et 3,35 ml (0,024 mole) de triéthylamine, on ajoute 5,07 g (0,022 mole) de chlorure de triméthoxy-3,4,5 benzoyle et porte à reflux sous agitation pendant 4 heures. Après refroidissement, on reprend le mélange avec 70 ml de chloroforme, lave successivement à l'eau, avec une solution de soude 2N et finalement à l'eau. Sèche sur sulfate de sodium. Le résidu obtenu, après évaporation du solvant, est recristallisé dans 8 volumes d'alcool. F=151°C.

C.C.M.: éluant C Rf=0,6
Analyse: $C_{22}H_{26}ClNO_7$ M=451,91

| | | | |
|---|---|---|---|
| Calc.%: | C: 58,47 | H: 5,80 | N 3,10 |
| Tr.%: | 58,47 | 5,68 | 3,11 |

Spectre I.R. (pastille KBr à 1%): bandes à 3260 (NH), 1730 (CO ester) et 1655 $cm^{-1}$ (CO amide).

## Exemple 10

*N-tert.-butyl (traans-cinnamoyloxy)-2 (diméthyl-2,6 phénoxy)-3 propanamide*
($A_4$; $R_1$=o—$CH_3$, $R_2$=o'—$CH_3$, $R_3$=$C_6H_5$—CH=CH, $R_4$=$(CH_3)_3C$, $R_5$=H) Composé No 58

A 5,3 g (0,02 mole) de N-tert.-butyl hydroxy-2 (diméthyl-2,6 phénoxy)-3 propanamide, 50 ml de toluène et 3,35 ml de triéthylamine, on ajoute 3,66 g (0,022 mole) de chlorure de trans-cinnamoyle et

chauffe au bain d'huile à 140°C pendant 4 heures. Après refroidissement, reprise au chloroforme et lavages comme dans l'exemple 9, le produit obtenu après l'évaporation est recristallisé dans 10 volumes d'éther isopropylique. F=108°C.

C.C.M.: éluant A Rf=0,6

Analyse: $C_{22}H_{29}NO_4$ M=395,5

| | | | |
|---|---|---|---|
| Calc. %: | C: 72,88 | H: 7,39 | N 3,54 |
| Trouvé%: | 72,53 | 7,42 | 3,57 |

Spectre I.R. (pastille KBr à 1%): bandes à 3260 (NH), 1720 (CO ester), 1670 (CO amide) et 1640 $cm^{-1}$ (double liaison).

Les amides des dérivés O-acylés des acides phénoxy lactiques (composés $A_4$) qui sont obtenus suivant les cas selon les exemples 6 (à partir des esters en position 2 des acides phénoxy lactiques), 9 (à partir des hydroxy amides $A_3$) ou 10 (à partir des hydroxy amides de Ritter $A_{3a}$) sont réunis dans le tableau IV.

TABLEAU IV : Acyloxy-2 phénoxy-3 propanamides :

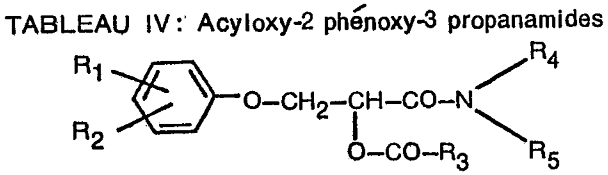

| Composé N° | $R_1$ | $R_2$ | $R_3$ | $R_4$ | $R_5$ | F °C | Recristallisation | C.C.M. Eluant | Rf |
|---|---|---|---|---|---|---|---|---|---|
| 26 | H | H | $CH_3$ | $C(CH_3)_3$ | H | 53 | Cyclohexane 5 vol. | B | 0,60 |
| 27 | H | H | $C_6H_5$ | $C(CH_3)_3$ | H | 141 | EtOH 4,5 vol. | B | 0,71 |
| 28 | H | H | p-$CH_3$–$C_6H_4$ | $C(CH_3)_3$ | H | 88 | Hexane 10 vol. | B | 0,78 |
| 29 | H | H | p-Cl–$C_6H_4$ | $C(CH_3)_3$ | H | 123 | Ether iso 15 vol. | B | 0,80 |
| 30 | H | H | $C_6H_5$–$CH_2$–$CH_2$ | $C(CH_3)_3$ | H | 57 | Ether de pétrole 3 vol. + cyclohexane 1,5 vol. | B | 0,80 |
| 31 | H | H | 3-pyridyl | $C(CH_3)_3$ | H | 112 | Ether iso 5 vol. + $CCl_4$ 2,5 vol. | C | 0,84 |
| 32 | H | H | $CH_3$ | $CH(CH_3)_2$ | H | 109 | $CCl_4$ 5 vol. + éther iso 14 vol. | B | 0,57 |
| 33 | H | H | $CH_3(CH_2)_{14}$ | $CH(CH_3)_2$ | H | 73 | EtOH 14 vol. | B | 0,66 |
| 34 | H | H | $C_6H_5$ | $CH(CH_3)_2$ | H | 139 | EtOH 5,5 vol. | B | 0,56 |
| 35 | H | H | p-$CH_3$–$C_6H_4$ | $CH(CH_3)_2$ | H | 133 | EtOH 5,5 vol. | B | 0,56 |
| 36 | H | H | p-$CH_3O$–$C_6H_4$ | $CH(CH_3)_2$ | H | 132 | EtOH 2,75 vol. + ether iso 2,75 vol. | B | 0,57 |
| 37 | H | H | p-Cl–$C_6H_4$ | $CH(CH_3)_2$ | H | 151 | MeOH 14 vol. | B | 0,67 |
| 38 | H | H | p-$NO_2$–$C_6H_4$ | $CH(CH_3)_2$ | H | 175 | AcOEt 11,5 vol. | non révélé | |
| 39 | H | H | p-$NH_2$–$C_6H_4$ | $CH(CH_3)_2$ | H | 173 | EtOH 20 vol. | B | 0,36 |
| 40 | H | H | 3,4,5$(CH_3O)_3C_6H_2$ | $CH(CH_3)_2$ | H | 145 | EtOH 5 vol. | B | 0,47 |

TABLEAU IV (Suite)

| Composé N° | $R_1$ | $R_2$ | $R_3$ | $R_4$ | $R_5$ | F °C | Recristallisation | C.C.M. Eluant | Rf |
|---|---|---|---|---|---|---|---|---|---|
| 41 | H | H | $C_6H_5-CH=CH$ | $CH(CH_3)_2$ | H | 134 | MeOH 4 vol. | B | 0,66 |
| 42 | H | H | $C_6H_5-CH_2-CH_2$ | $CH(CH_3)_2$ | H | 73 | Ether iso 5 vol. | B | 0,58 |
| 43 | H | H | 3-pyridyl | $CH(CH_3)_2$ | H | 143 | AcOEt 7 vol. | B | 0,21 |
| 44 | H | H | $CH_3$ | $C_6H_5$ | H | 81 | Ether iso 10 vol. | B | 0,67 |
| 45 | H | H | $CH_3$ | $(CH_2)_2-O-(CH_2)_2$ | | 89 | $CCl_4$ 1 vol. + ether iso 9 vol. | B | 0,48 |
| 46 | o-$CH_3$ | H | $3,4,5(CH_3O)_3C_6H_2$ | $C(CH_3)_3$ | H | 129 | éther iso 20 vol. | C | 0,95 |
| 47 | o-$CH_3$ | H | $C_6H_5-CH=CH$ | $C(CH_3)_3$ | H | 86 | iso-PrOH 4 vol. | B | 0,71 |
| 48 | p-$CH_3$ | H | $CH_3$ | $C(CH_3)_3$ | H | 76 | éther iso 5 vol. | A | 0,51 |
| 49 | p-$CH_3$ | H | p-$CH_3-C_6H_4$ | $C(CH_3)_3$ | H | 106 | éther iso 10 vol. | A | 0,65 |
| 50 | p-$CH_3$ | H | p-$Cl-C_6H_4$ | $C(CH_3)_3$ | H | 142 | EtOH 6 vol. | A | 0,62 |
| 51 | p-$CH_3$ | H | p-$NO_2-C_6H_4$ | $C(CH_3)_3$ | H | 158 | EtOH 18 vol. | A | 0,54 |
| 52 | p-$CH_3$ | H | p-$NH_2-C_6H_4$ | $C(CH_3)_3$ | H | 122 | EtOH 2 vol. + hexane 15 vol. | C | 0,42 |
| 53 | p-$CH_3$ | H | $3,4,5(CH_3O)_3C_6H_2$ | $C(CH_3)_3$ | H | 97 | éther iso 11,5 vol. | A | 0,43 |
| 54 | p-$CH_3$ | H | $C_6H_5-CH=CH$ | $C(CH_3)_3$ | H | 125 | EtOH 5 vol. | A | 0,59 |
| 55 | p-$CH_3$ | H | 3-pyridyl | $C(CH_3)_3$ | H | 132 | EtOH 1,5 vol. + éther iso 24,5 vol. | A | 0,39 |
| 56 | o-$CH_3$ | o'-$CH_3$ | $CH_3(CH_2)_{14}$ | $C(CH_3)_3$ | H | 80 | Iso-PrOH | A | 0,61 |
| 57 | o-$CH_3$ | o'-$CH_3$ | $3,4,5(CH_3O)_3C_6H_2$ | $C(CH_3)_3$ | H | 112 | Iso-PrOH 5 vol. | A | 0.46 |

TABLEAU IV (Suite)

| Composé N° | $R_1$ | $R_2$ | $R_3$ | $R_4$ | $R_5$ | F°C | Recristallisation | C.C.M. Eluant | Rf |
|---|---|---|---|---|---|---|---|---|---|
| 58 | o-$CH_3$ | o'-$CH_3$ | $C_6H_5-CH=CH$ | $C(CH_3)_3$ | H | 108 | éther iso 10 vol. | A | 0,60 |
| 59 | o-$CH_3$ | o'-$CH_3$ | 3-pyridyl | $C(CH_3)_3$ | H | 125 | éther iso 15 vol. | A | 0,35 |
| 60 | o-$CH_3O$ | H | $CH_3$ | $C(CH_3)_3$ | H | 88 | éther iso 6 vol. | B | 0,53 |
| 61 | o-$CH_3O$ | H | $Cl-CH_2$ | $C(CH_3)_3$ | H | 103 | éther iso 11,5 vol. | B | 0,60 |
| 62 | o-$CH_3O$ | H | $CH_3-(CH_2)_{14}$ | $C(CH_3)_3$ | H | 66 | hexane 5 vol. | B | 0,67 |
| 63 | o-$CH_3O$ | H | $C_6H_5$ | $C(CH_3)_3$ | H | 135 | MeOH 4 vol. | C | 0,44 |
| 64 | o-$CH_3O$ | H | p-$CH_3-C_6H_4$ | $C(CH_3)_3$ | H | 137 | EtOH 6 vol. | B | 0,64 |
| 65 | o-$CH_3O$ | H | p-$CH_3O-C_6H_4$ | $C(CH_3)_3$ | H | 136 | iso-PrOH 5 vol. | B | 0,53 |
| 66 | o-$CH_3O$ | H | p-$Cl-C_6H_4$ | $C(CH_3)_3$ | H | 154 | EtOH 7 vol. | B | 0,60 |
| 67 | o-$CH_3O$ | H | p-$NO_2-C_6H_4$ | $C(CH_3)_3$ | H | 144 | EtOH 7 vol. | B | 0,79 |
| 68 | o-$CH_3O$ | H | p-$NH_2-C_6H_4$ | $C(CH_3)_3$ | H | 145 | EtOH 7 vol. | B | 0,41 |
| 69 | p-$CH_3O$ | H | 3,4,5$(CH_3O)_3C_6H_2$ | $C(CH_3)_3$ | H | 113 | éther iso 20 vol. | B | 0,79 |
| 70 | o-$CH_3O$ | H | $C_6H_5-CH=CH$ | $C(CH_3)_3$ | H | 152 | AcOEt 7 vol. | B | 0,77 |
| 71 | o-$CH_3O$ | H | $C_6H_5-CH_2-CH_2$ | $C(CH_3)_3$ | H | 75 | hexane 11,5 vol. | B | 0,53 |
| 72 | o-$CH_3O$ | H | 3-pyridyl | $C(CH_3)_3$ | H | 98 | éther iso 9 vol. | C | 0,73 |
| 73 | o-$CH_3O$ | H | $CH_3$ | $CH(CH_3)_2$ | H | 87 | digestion dans hexane | C | 0,78 |
| 74 | p-$CH_3O$ | H | $CH_3$ | $C(CH_3)_3$ | H | 75 | éther iso 3 vol. + cyclohexane 1 vol. | C | 0,45 |
| 75 | p-$CH_3O$ | H | $Cl-CH_2$ | $C(CH_3)_3$ | H | 93 | éther iso 6 vol. | C | 0,45 |

TABLEAU IV (Suite)

| Composé N° | $R_1$ | $R_2$ | $R_3$ | $R_4$ | $R_5$ | F°C | Recristallisation | C.C.M. Eluant | Rf |
|---|---|---|---|---|---|---|---|---|---|
| 76 | p-$CH_3O$ | H | $CH_3-(CH_2)_{14}$ | $C(CH_3)_3$ | H | 55 | MeOH 3 vol. | C | 0,81 |
| 77 | p-$CH_3O$ | H | $C_6H_5$ | $C(CH_3)_3$ | H | 124 | EtOH 1 vol.<br>éther iso 5 vol | C | 0,45 |
| 78 | p-$CH_3O$ | H | p-$CH_3-C_6H_4$ | $C(CH_3)_3$ | H | 90 | iso-PrOH 3 vol. | C | 0,45 |
| 79 | p-$CH_3O$ | H | p-$CH_3O-C_6H_4$ | $C(CH_3)_3$ | H | 128 | MeOH 5 vol. | B | 0,60 |
| 80 | p-$CH_3O$ | H | p-$Cl-C_6H_4$ | $C(CH_3)_3$ | H | 110 | MeOH 7 vol. | C | 0,50 |
| 81 | p-$CH_3O$ | H | p-$NO_2-C_6H_4$ | $C(CH_3)_3$ | H | 110 | EtOH 10 vol. | C | 0,55 |
| 82 | p-$CH_3O$ | H | p-$NH_2-C_6H_4$ | $C(CH_3)_3$ | H | 151 | MeOH 7 vol. | C | 0,36 |
| 83 | p-$CH_3O$ | H | $3,4,5(CH_3O)_3C_6H_2$ | $C(CH_3)_3$ | H | 131 | MeOH 7 vol. | C | 0,43 |
| 84 | p-$CH_3O$ | H | $C_6H_5-CH=CH$ | $C(CH_3)_3$ | H | 130 | MeOH 7 vol. | C | 0,43 |
| 85 | p-$CH_3O$ | H | $C_6H_5-CH_2-CH_2$ | $C(CH_3)_3$ | H | 95 | MeOH 4 vol. | C | 0,53 |
| 86 | p-$CH_3O$ | H | 3-pyridyl | $C(CH_3)_3$ | H | 116 | EtOH 1 vol. +<br>éther iso 5 vol. | C | 0,29 |
| 87 | p-Cl | H | $CH_3$ | $C(CH_3)_3$ | H | 104 | cyclohexane 5 vol. | D | 0,95 |
| 88 | p-Cl | H | $Cl-CH_2$ | $C(CH_3)_3$ | H | 96 | iso-PrOH 3 vol. | C | 0,59 |
| 89 | p-Cl | H | $CH_3-(CH_2)_{14}$ | $C(CH_3)_3$ | H | 60 | éther iso 5 vol. | C | 0,73 |
| 90 | p-Cl | H | $C_6H_5$ | $C(CH_3)_3$ | H | 152 | éther iso | B | 0,69 |
| 91 | p-Cl | H | p-$CH_3-C_6H_4$ | $C(CH_3)_3$ | H | 105 | iso-PrOH 4 vol. | D | 0,96 |
| 92 | p-Cl | H | p-$CH_3O-C_6H_4$ | $C(CH_3)_3$ | H | 139 | EtOH 9,5 vol. | B | 0,80 |
| 93 | p-Cl | H | p-$Cl-C_6H_4$ | $C(CH_3)_3$ | H | 152 | EtOH 12 vol. | C | 0,66 |

TABLEAU IV (Suite)

| Composé N° | $R_1$ | $R_2$ | $R_3$ | $R_4$ | $R_5$ | F°C | Recristallisation | C.C.M. Eluant | Rf |
|---|---|---|---|---|---|---|---|---|---|
| 94 | p-Cl | H | p-$NO_2$–$C_6H_4$ | $C(CH_3)_3$ | H | 157 | EtOH 12 vol. | B | 0,50 |
| 95 | p-Cl | H | p-$NH_2$–$C_6H_4$ | $C(CH_3)_3$ | H | 158 | EtOH 10 vol. | B | 0,56 |
| 96 | p-Cl | H | 3,4,5$(CH_3O)_3C_6H_2$ | $C(CH_3)_3$ | H | 118 | éther iso 8 vol. | C | 0,86 |
| 97 | p-Cl | H | $C_6H_5$–CH=CH | $C(CH_3)_3$ | H | 128 | cyclohexane 9 vol. | D | 0,97 |
| 98 | p-Cl | H | $C_6H_5$–$CH_2$–$CH_2$ | $C(CH_3)_3$ | H | 62 | hexane 5 vol. | B | 0,63 |
| 99 | p-Cl | H | 3-pyridyl | $C(CH_3)_3$ | H | 143 | EtOH 2 vol. + éther iso 2 vol. | C | 0,39 |
| 100 | p-Cl | H | $CH_3$ | $CH(CH_3)_2$ | H | 108 | EtOH 7,5 vol. + eau 7,5 vol. | C | 0,59 |
| 101 | p-Cl | H | Cl–$CH_2$ | $CH(CH_3)_2$ | H | 88 | éther iso 6 vol. | C | 0,61 |
| 102 | p-Cl | H | $(CH_3)_2CH$–$CH_2$ | $CH(CH_3)_2$ | H | 76 | cyclohexane 5 vol. | C | 0,54 |
| 103 | p-Cl | H | $CH_3$–$(CH_2)_{14}$ | $CH(CH_3)_2$ | H | 74 | EtOH 5 vol. | C | 0,68 |
| 104 | p-Cl | H | $C_6H_5$ | $CH(CH_3)_2$ | H | 144 | EtOH 10 vol. | C | 0,63 |
| 105 | p-Cl | H | p-$CH_3$–$C_6H_4$ | $CH(CH_3)_2$ | H | 138 | EtOH 5 vol. | C | 0,76 |
| 106 | p-Cl | H | p-$CH_3O$–$C_6H_4$ | $CH(CH_3)_2$ | H | 136 | EtOH 6 vol. | C | 0,53 |
| 107 | p-Cl | H | p-Cl–$C_6H_4$ | $CH(CH_3)_2$ | H | 176 | AcOEt 12 vol. | C | 0,70 |
| 108 | p-Cl | H | p-$NO_2$–$C_6H_4$ | $CH(CH_3)_2$ | H | 182 | AcOH 7 vol. | C | 0,46 |
| 109 | p-Cl | H | p-$NH_2$–$C_6H_4$ | $CH(CH_3)_2$ | H | 150 | EtOH 7 vol. | C | 0,38 |
| 110 | p-Cl | H | 3,4,5$(CH_3O)_3C_6H_2$ | $CH(CH_3)_2$ | H | 151 | EtOH 8 vol. | C | 0,62 |
| 111 | p-Cl | H | $C_6H_5$–CH=CH | $CH(CH_3)_2$ | H | 136 | EtOH 5 vol. | C | 0,70 |

TABLEAU IV (Suite)

| Composé N° | $R_1$ | $R_2$ | $R_3$ | $R_4$ | $R_5$ | F°C | Recristallisation | C.C.M. Eluant | Rf |
|---|---|---|---|---|---|---|---|---|---|
| 112 | p-Cl | H | $C_6H_5-CH_2-CH_2$ | $CH(CH_3)_2$ | H | 111 | EtOH 8 vol. | C | 0,66 |
| 113 | p-Cl | H | 3-pyridyl | $CH(CH_3)_2$ | H | 160 | EtOH 6 vol. | C | 0,33 |
| 114 | p-Cl | H | $CH_3$ | $(CH_2)_2-O-(CH_2)_2$ | | 77 | cyclohexane 9 vol. | C | 0,45 |
| 115 | p-Cl | H | $CH_3$ | $C_6H_5$ | H | 102 | éther iso 7 vol. | C | 0,55 |

**Revendications**

1. Procédé de fabrication des acides phénoxylactiques et de leurs dérivés de formule générale:

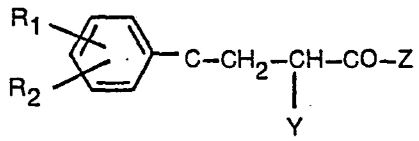

($A_1$)

(dans laquelle $R_1$ et $R_2$, identiques ou différents, peuvent représenter des atomes d'hydrogène, des restes méthyle, des groupes méthoxy ou des halogènes; Y représente un groupement —OH ou —O—CO—$R_3$ avec $R_3$ représentant soit un reste aliphatique éventuellement substitué par un halogène, soit un reste aromatique éventuellement substitué sur le noyau par un ou plusieurs groupements méthyle, méthoxy, halogéno, soit enfin un hétérocycle; Z représente le groupement OH ou un groupement

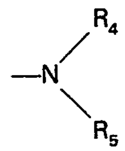

avec $R_4$ représentant un radical alkyle ou aryle et

$R_5$ de l'hydrogène ou un radical alkyle, $NR_4R_5$ pouvant aussi représenter le radical morpholinyle), procédé caractérisé par le fait qu'il consiste à soumettre une cyanhydrine de formule:

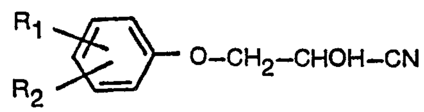

(A)

(où $R_1$ et $R_2$ ont la signification ci-dessus) à une réaction de RITTER (réaction avec du tert.butanol ou de l'isobutène en présence d'un acide) pour obtenir le composé de formule:

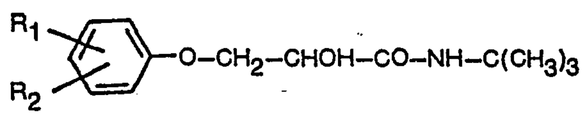

($A_{3a}$)

et à hydrolyser ce dernier pour obtenir l'acide correspondant de formule ($A_1$) telle que définie ci-dessus, dans laquelle Z et Y représentent —OH, puis, si on le désire, à soumettre cet acide aux réactions classiques d'obtention des esters-acides, esters-amides ou amides correspondants respectivement de formule:

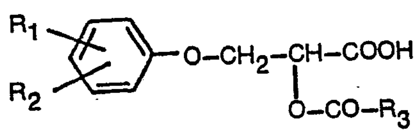

($A_2$)

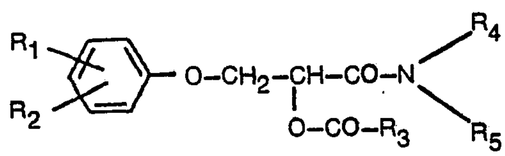

($A_4$)

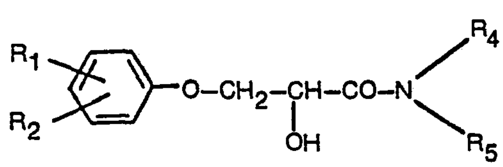

($A_3$)

(où $R_1$ et $R_2$, $R_3$, $R_4$ et $R_5$ ont la signification ci-dessus).

2. Acides acétoxy-2 (p-chlorophénoxy)-3-propanoïque et acétoxy-2 phénoxy-3 propanoïque

3. N-tert-butyl hydroxy-2 phénoxy-3 propanamide N-tert-butyl hydroxy-2 (o-tolyloxy)-3 propanamide N-tert-butyl hydroxy-2 (p-tolyloxy)-3 propanamide N-tert-butyl hydroxy-2 (diméthyl-2,6 phénoxy)-3 propanamide

N-tert-butyl hydroxy-2 (o-méthoxy phénoxy)-3 propanamide

N-tert-butyl hydroxy-2 (p-méthoxy phénoxy)-3 propanamide

N-tert-butyl hydroxy-2 (p-chloro phénoxy)-3 propanamide
N-isopropyl hydroxy-2 (p-chloro-phénoxy)-3 propanamide
N-isopropyl hydroxy-2 phénoxy-3 propanamide

4. Acyloxy-2 phenoxy-3 propanamides de formule générale:

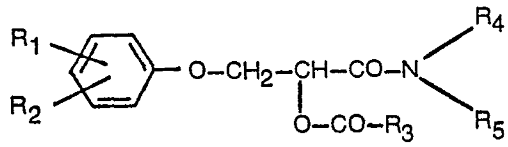

dans laquelle:

—$R_1$ et $R_2$ sont tous les deux des atomes d'hydrogène;

—ou bien $R_1$ est un radical o-méthyle, p-méthyle, o-méthoxy, p-méthoxy ou p-chloro et $R_2$ est un atome d'hydrogène;

—ou bien $R_1$ est un radical o-méthyle et $R_2$ un radical o'-méthyle;

—$R_3$ est un radical méthyle, chlorométhyle, iso-propyle, n-pentadécyle, phényle, p-tolyle, p-méthoxyphényle, p-chloro-phényle, p-nitro phényle, p-aminophényle, 3,4,5-triméthoxyphényle, 2-phenyléthényl, 2-phényléthyl, 3-pyridyl;

—$R_4$ est un radical isopropyle, tert-butyle, phényle et $R_5$ est un atome d'hydrogène;

—ou bien $R_4$ et $R_5$ ensemble forment avec l'atome d'azote auquel ils sont liés un radical morpholinyle.

## Claims

1. A process for preparation of phenoxylactic acids and their derivatives of the general formula:

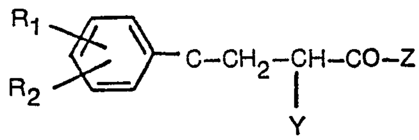

($A_1$)

(in which $R_1$ and $R_2$, identical or different, may represent hydrogen atoms, methyl rests, methoxy groups or halogens; Y represents an OH or O—CO—$R_3$ group with $R_3$ representing either an aliphatic rest, possibly substituted by a halogen or an aromatic rest, possibly substituted on the ring by one or more methyl, methoxy, halogeno groups, or finally, a heterocycle; z represents the OH group or an

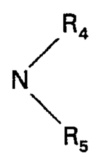

group, with $R_4$ representing an alkyl or aryl radical, and $R_5$ hydrogen or an alkyl radical, $NR_4R_5$ being capable of also representing the morpholinyl radical), the process being characterized in that it consists of submitting a cyanohydrin of the formula:

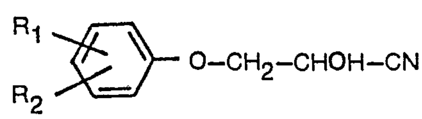

(A)

(wherein $R_1$ and $R_2$ have the above meaning) to a RITTER reaction (reaction with tert.butanol or isobutene in the presence of an acid) to obtain the compound of the formula:

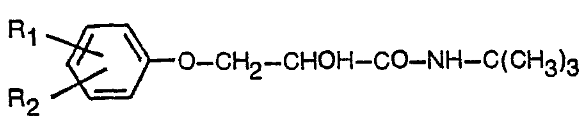

($A_{3a}$)

and hydrolyzing the latter to obtain the corresponding acid of formula ($A_1$) such as defined above, in which Z and Y represent —OH, then, if desired, submitting such acid to the conventional reactions for obtaining the corresponding esters acids, esters amides or amides, respectively of the formula:

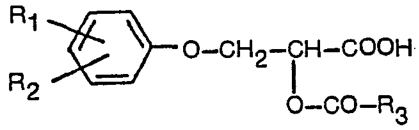

$(A_8)$

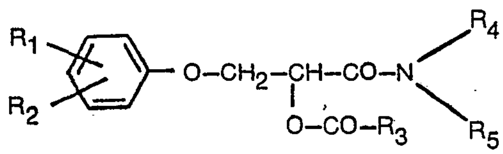

$(A_4)$

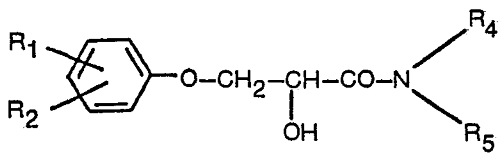

$(A_3)$

(wherein $R_1$ and $R_2$, $R_3$, $R_4$ and $R_5$ have the above-mentioned meaning).

2. 2-acetoxy-3-(p-chlorophenoxy)-propanoic and 2-acetoxy-3-phenoxy-propanioc acids.

3. N-tert-butyl-2-hydroxy-3-phenoxy) propanamide,
N-tert-butyl-2-hydroxy-3(o-tolyloxy) propanamide,
N-tert-butyl-2-hydroxy-3(p-tolyloxy) propanamide,
N-tert-butyl-2-hydroxy-3(2,6-dimethyl-phenoxy) propanamide,
N-tert-butyl-2-hydroxy-3(o-methoxy-phenoxy) propanamide,
N-tert-butyl-2-hydroxy-3(p-methoxy-phenoxy) propanamide,
N-tert-butyl-2-hydroxy-3(p-chloro-phenoxy) propanamide,
N-isopropyl-2-hydroxy-3(p-chloro-phenoxy) propanamide,
N-isopropyl-2-hydroxy-3-phenoxy propanamide.

4. 2-acyloxy-3-phenoxy propanamides of the general formula:

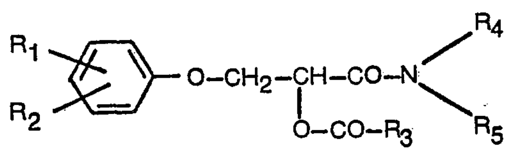

in which:
—$R_1$ and $R_2$ are both hydrogen atoms;
—or $R_1$ is an o-methyl, p-methyl, o-methoxy, p-methoxy, or p-chloro radical and $R_2$ is a hydrogen atom;
—or $R_1$ is an o-methyl radical and $R_2$ an o'-methyl radical;
—$R_3$ is a methyl, chloromethyl, isopropyl, n-pentadecyl, phenyl, p-tolyl, p-methoxyphenyl, p-chlorophenyl, p-nitro-phenyl, p-aminophenyl, 3,4,5-trimethoxyphenyl, 2-phenylethenyl, 2-phenylethyl, 3-pyridyl radical;
—$R_4$ is an isopropyl, tert-butyl, phenyl radical, and $R_5$ is a hydrogen atom;
—or $R_4$ and $R_5$ together form with the nitrogen atom to which they are bonded a morpholinyl radical.

**Patentansprüche**

1. Verfahren zur Herstellung von Phenoxyessigsäuren und deren Derivaten der allgemeinen Formel

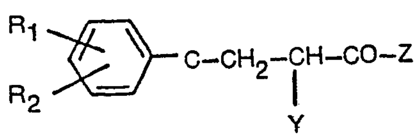

$A_1$

worin $R_1$ und $R_2$, die gleich oder verschieden sein können, Wasserstoffatome, Methylgruppen, Methoxygruppen oder Halogen bedeuten, Y die Gruppe OH oder O—CO—$R_3$ darstellt, wobei $R_3$ einen, gegebenenfalls halogensubstituierten, aliphatischen Rest oder einen, gegebenenfalls mit einem oder mehreren Methyl- oder Methoxy- oder Halogenresten kernsubstituierten, aromatischen Rest oder einen Heterozyklus bedeutet, Z die Gruppe OH oder die Gruppe

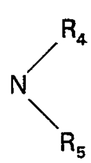

darstellt, wobei $R_4$ einen Alkyl- oder Arylrest und $R_5$ Wasserstoff oder einen Alkylrest bedeuten, und $NR_4R_5$ auch einen Morpholinylrest darstellen kann, dadurch gekennzeichnet, dass man ein Cyanhydrin der Formel

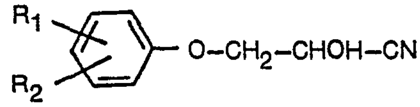

A

worin $R_1$ und $R_2$ die oben angegebenen Bedeutungen besitzen, einer Reaktion nach RITTER (Umsetzung mit tert.-Butanol oder Isobuten in Gegenwart einer Säure) unterwirft, um die Verbindung der Formel

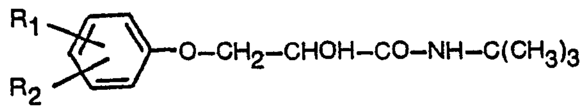

$A_{3a}$

zu erhalten, und diese letztere Verbindung hydrolisiert um die entsprechende Säure der Formel A1, wie sie oben definiert ist, zu erhalten, worin Z und Y die Gruppe —OH darstellen, und dann gewünschtenfalls diese Säure einer klassischen Reaktion zur Erzielung der entsprechenden Ester-Säuren, Ester-Amide oder Amide der Formel

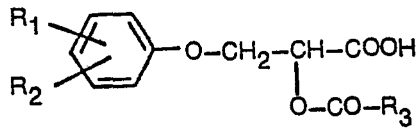

$A_2$

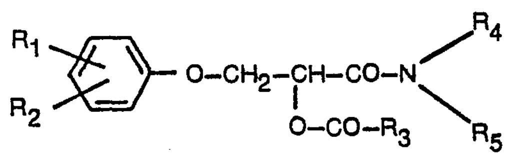

$A_4$

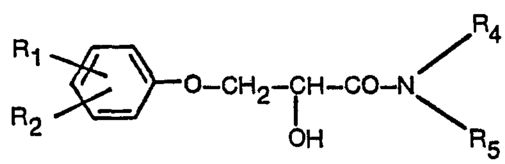

$A_3$

unterwirft, worin $R_1$ und $R_2$, $R_3$, $R_4$ und $R_5$ die oben angegebenen Bedeutungen besitzen.

2. 2-Acetoxy-3-(p-chlorophenoxy)-propansäure und 2-Acetoxy-3-phenoxy-propansäure.

3. N-tert-Butyl-2-hydroxy-3-phenoxy-propanamid
N-tert-Butyl-2-hydroxy-3-(o-tolyloxy)-propanamid
N-tert-Butyl-2-hydroxy-3-(p-tolyloxy)-propanamid
N-tert-Butyl-2-hydroxy-3-(2,6-dimethyl-phenoxy)-propanamid
N-tert-Butyl-2-hydroxy-3-(o-methoxy-phenoxy)-propanamid
N-tert-Butyl-2-hydroxy-3-(p-methoxy-phenoxy)-propanamid
N-tert-Butyl-2-hydroxy-3-(p-chlor-phenoxy)-propanamid
N-Isopropyl-2-hydroxy-3-(p-chlor-phenoxy)-propanamid
N-Isopropyl-2-hydroxy-3-phenoxy-propanamid.

4. 2-Acyloxy-3-phenoxy-propanamide der allgemeinen Formel

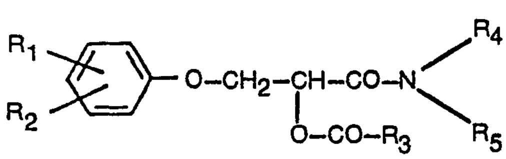

worin $R_1$ und $R_2$ beide Wasserstoffatome bedeuten oder $R_1$ ein o-Methyl-, p-Methyl-, o-Methoxy-, p-Methoxy- oder p-Chlorrest und $R_2$ ein Wasserstoffatom bedeuten; oder worin $R_1$ einen o-Methylrest und $R_2$ einen o-Methylrest bedeuten;
$R_3$ einen Methyl-, Chlormethyl-, Isopropyl-, n-Pentadecyl-, Phenyl-, p-Tolyl-, p-Methoxyphenyl-, p-Chlorphenyl-, p-Nitrophenyl-, p-Aminophenyl-, 3,4,5-Trimethoxyphenyl-, 2-Phenylethenyl-, 2-Phenylethyl- oder 3-Pyridylrest bedeutet;
$R_4$ einen Isopropyl-, tert-Butyl- oder Phenylrest und $R_5$ ein Wasserstoffatom bedeuten, oder worin $R_4$ und $R_5$ gemeinsam mit dem Stickstoffatom, an das sie gebunden sind, einen Morpholinylrest darstellen.